# FASCICULE DE BREVET EUROPEEN

(11) **EP 1 986 502 B1**
(45) Date de publication et mention de la délivrance du brevet: **22.03.2017**
(21) Numéro de dépôt: 07717991.9
(22) Date de dépôt: 17.01.2007
(51) Int. Cl.: A23C 19/032, A23C 9/12, C12R 1/13, C12R 1/225, A23C 19/05, A23C 19/072, A23C 20/02, C12N 1/20, C12P 39/00

(54) **COMPOSITION ET PROCEDE D'AROMATISATION DE PRODUITS LAITIERS, SOUCHE DE BACTERIE LACTIQUE, UTILISATION DESDITES COMPOSITION OU SOUCHE**
ZUSAMMENSETZUNG UND VERFAHREN FÜR DIE AROMATISIERUNG VON MILCHPRODUKTEN, MILCHSÄUREBAKTERIUMSTAMM, VERWENDUNG DER ZUSAMMENSETZUNG BZW. DES STAMMS
COMPOSITION AND PROCESS FOR FLAVOURING DAIRY PRODUCTS, STRAIN OF LACTIC ACID BACTERIUM, USE OF SAID COMPOSITION OR STRAIN

(30) Priorité: 20.01.2006 FR 0600546
(43) Date de publication de la demande: 05.11.2008
(73) Titulaire: DuPont Nutrition Biosciences ApS, 1411 Copenhagen K (DK)
(72) Inventeur: MORNET, Annie, F-86230 MONDION (FR); GOODWINS, Jonathan, F-37160 Abilly (FR)
(74) Mandataire: DuPont EMEA
(86) Numéro de dépôt international: PCT/FR2007/000085
(87) Numéro de publication internationale: WO 2007/083021

(56) Documents cités:
- EP-A1- 0 058 856
- EP-A2- 0 384 553
- VALENCE FLORENCE ET AL: "Autolysis and related proteolysis in Swiss cheese for two Lactobacillus helveticus strains" JOURNAL OF DAIRY RESEARCH, vol. 67, no. 2, mai 2000 (2000-05), pages 261-271, XP009073822 ISSN: 0022-0299
- DATABASE WPI Week 200567 Derwent Publications Ltd., London, GB; AN 2005-655305 XP002405017 & RU 2 260 978 C2 (BESSEREZHNOV A S) 27 septembre 2005 (2005-09-27)

## Description

La présente invention concerne l'industrie laitière, en particulier la fabrication de produits laitiers, et plus particulièrement la fabrication de fromages, notamment la fabrication de fromage à pâte pressée non cuite.

La fabrication des produits laitiers nécessite l'utilisation de différents microorganismes ayant des rôles spécifiques en technologie alimentaire.

Notamment, les bactéries lactiques sont utilisées dans l'acidification du lait ce qui provoque la fermentation du lait en caillé. Les bactéries lactiques sont introduites dans le lait à fermenter sous formes de ferments, appelés également ferments d'acidification, cultures starter ou starters.

D'autres microorganismes sont quant à eux utilisés pour l'affinage des fromages. Dans ce cas, l'on parle de flore d'affinage, ou agents d'affinage ou encore de non-starters. Ces microorganismes permettent de transformer le caillé de lait en un produit partiellement lipolysé, protéolysé, enrichi en composé en composés aromatiques. Les composés aromatiques sont les composés développant la flaveur du fromage. Ils sont constitués par exemple d'acides aminés, d'acides gras, de peptides aromatiques ou de composés volatiles tels que des esters, des dérivés volatiles cétoniques ou soufrés etc... L'on entend par flaveur dans le présent document, l'ensemble des sensations olfactives, gustatives et trigéminales perçues au cours de la dégustation.

Les agents d'affinage couramment utilisés sont des bactéries par exemple les bactéries du genre *Arthrobacter, Corynebacterium, Lactobacillus, Lactococcus, Leuconostoc, Micrococcus, Pediococcus, Propionibacterium, Staphylococcus* et *Streptococcus.* Ils peuvent également être des levures ou des moisissures comme par exemple les microorganismes du genre *Candida, Debariomyces, Geotrichum, Kluyveromyces, Rhodotorula, Saccharomyces ou Penicilium.*

L'affinage est d'une grande importance pour donner de la flaveur au produit laitier.

Or l'addition d'agents d'affinage traditionnels aux ferments d'acidification est insuffisante dans certains cas pour développer la flaveur souhaitée dans le produit laitier. En effet, les agents d'affinage traditionnels ne permettent pas d'aromatiser le produit laitier suffisamment, notamment lorsque la durée d'affinage est courte ou réduite. Dans ce cas, le profil aromatique recherché n'est pas obtenu et d'autres flaveurs indésirables comme par exemple l'amertume, peuvent apparaître.

Afin de répondre aux exigences des consommateurs et des industriels, il est devenu indispensable de trouver de nouveaux microorganismes susceptibles d'affiner et d'aromatiser les produits laitiers.

Par ailleurs, l'utilisation de microorganismes du genre *Lactobacillus* de type non lytique est connue.

En outre, on connaît l'utilisation agents d'affinage du genre *Brevibacterium* pour l'aromatisation de fromage.

Cependant, l'utilisation combinée de ces deux souches ne permet d'obtenir un affinage et une aromatisation des produits laitiers pouvant répondre aux exigences susmentionnées.

Le document EP 0 384 553 A2 décrit l'utilisation d'une souche *de L. helveticus,* d'une souche de *Brevibacterium linens,* d'une souche de *Geotrichum candidum* et d'une souche de *Debaromyces hansenii* pour la fabrication d'un fromage de type « Tilsit ».

Le document RU 2 260 978 décrit un ferment comprenant, entre autres, *L*. *helveticus et B linens.*

Le document Valence et al., Journal of Dairy Research, vol 67, p 261-271, (2000), décrit des souches de L helveticus ayant différentes propriétés lytiques.

L'invention a pour but de proposer un nouveau moyen pour aromatiser des produits laitiers qui présente de nombreuses qualités et qui permet de développer la flaveur souhaitée aux produits laitiers et cela de manière significative, rapide et permanente.

A cet effet, l'invention a pour objet une composition d'aromatisation de produits laitiers, notamment de fromages, comprenant au moins une combinaison de microorganismes, ladite combinaison comprenant au moins microorganisme de type lytique et au moins un agent d'affinage, telle que définie dans les revendications.

L'on entend par microorganisme de type lytique, toute bactérie d'acidification ou d'affinage présentant des propriétés enzymatiques élevées. En particulier, dans le cas présent, ce sont des microorganisme qui libèrent, par autolyse, au bout d'une durée classique d'affinage, 40 % ou plus de leurs enzymes protéolytiques intracellulaires dans leur milieu environnant en condition classique d'utilisation (c'est-à-dire dans des conditions de contraintes physiques, de salinité et de pH semblables à celle présentes dans un produit laitier).

Typiquement pour mesurer le % d'enzymes protéolytiques intracellulaires libérées dans le milieu environnant par autolyse, l'homme du métier pourra comparer l'activité intracellulaire de la dipeptidase PepD d'une quantité déterminée de microorganismes et la comparer à l'activité enzymatique de la dipeptidase PepD libérée par la même quantité de microorganismes dans le milieu environnant au bout de 24 heures à 40°C dans un milieu tamponné à un pH de 5,8 par tampon phosphate de potassium à 0,1 M.

Les agents d'affinage sont les bactéries du genre *Arthrobacter, Brevibacterium' Corynebacterium, Lactobacillus, Lactococcus, Leuconostoc, Micrococcus, Pediococcus, Propionibacterium, Staphylococcus* et *Streptococcus.* D'autres agents d'affinage connus sont des levures ou des moisissures comme par exemple les microorganismes du genre *Candida, Debariomyces, Geotrichum, Kluyveromyces, Rhodotorula*, *Saccharomyces ou Penicilium.*

En effet, l'utilisation de microorganismes de type lytique en combinaison avec au moins un agent d'affinage pour l'affinage de produits laitiers permet d'une part de potentialiser, c'est-à-dire, d'augmenter voire de renforcer, le développement de la flaveur par rapport à une utilisation de microorganismes de flore d'affinage classique.

Typiquement, le rapport en nombre microorganismes de type lytique sur agents d'affinage pourra être compris entre 30/70 et 70/30, preférentiellement le rapport pourra être compris entre 40/60 et 60/40.

Cette synergie se manifeste non seulement par l'obtention d'une quantité significativement plus élevée de composés d'aromatisation classiques dans le fromage tels que ceux du type diacétyle, 2-butanone, acétoïne, DMDS (disulfure de diméthyl), 1-octén-3-ol, DMTS (trisulfure de diméthyl) et acide butyrique.

L'action de microorganismes de type lytique en combinaison avec un agent d'affinage permet en outre, par synergie avec ledit agent d'affinage, de promouvoir le développement de la flaveur par ledit agent d'affinage. Il permet notamment à *Brevibacterium linens* de produire davantage de composés soufrés.

De plus, l'utilisation de microorganismes de type lytique pour l'affinage de produits laitiers permet de diminuer la proportion de composés responsables de la flaveur de type amère.

Enfin, l'utilisation de microorganismes de type lytique en combinaison avec un agent d'affinage permet la production de peptides de petite taille (inférieure à 1kDa), hydrophiles, qui ne sont pas produits lorsque les différentes souches sont cultivées indépendamment. Cette synergie permet donc la production de peptides différents qui confèrent des nouveaux goûts (par exemple du type bouillon de viande, umami, savoureux) au produit laitier fini.

Un autre avantage de la présente invention est que le microorganisme de type lytique peut être utilisé conjointement avec des bactéries utilisées pour l'acidification de produits laitiers, c'est-à-dire avec les ferments d'acidification (par exemple du type *Lactococcus lactis* et/ou *Streptococcus thermophilus*).

Avantageusement, ledit agent d'affinage est un microorganisme du genre *Arthrobacter* ou *Brevibacterium,* en particulier du genre *Brevibacterium linens.*

L'utilisation de la combinaison de microorganismes du genre *Lactobacillus helveticus* de type lytique et de microorganisme du genre *Brevibacterium* permet de renforcer le développement de flaveur par les microorganismes du genre *Brevibacterium.*

Selon la présente invention, ledit microorganisme de type lytique est la souche *Lactobacillus helveticus* LbH 210 déposée au nom de Danisco France SAS, 20 rue de Brunel, 75017 Paris, à la CNCM (Collection Nationale de Culture de Microorganismes) le 20 janvier 2006 sous le numéro CNCM 1-3554.

Dans un autre mode de réalisation avantageux, ledit agent d'affinage est la souche *Brevibacterium* 3306 déposée au nom de Danisco France SAS, 20 rue de Brunel, 75017 Paris, à la CNCM le 20 janvier 2006 sous le numéro CNCM 1-3556.

Dans un autre mode de réalisation avantageux, ledit agent d'affinage est la souche *Brevibacterium* 3383 déposée au nom de Danisco France SAS, 20 rue de Brunel, 75017 Paris, à la CNCM le 20 janvier 2006 sous le numéro CNCM 1-3555.

Dans un mode de réalisation particulier, lesdits microorganismes de type lytique et lesdits agents d'affinage sont en mélange ou en forme séparée.

Avantageusement, lesdits microorganismes de type lytique et lesdits agents d'affinage sont sous forme d'un mélange de souches lyophilisé ou congelé, ou sous forme de souches conditionnées de manière séparée.

La présente invention a également pour objet une souche *Lactobacillus helveticus* LbH 210 déposée au nom de Danisco France SAS, 20 rue de Brunel, 75017 Paris, à la CNCM le 20 janvier 2006 sous le n° CNCM 1-3554.

Cette souche permet de potentialiser la flaveur développée par les agents d'affinage d'un produit laitier.

La présente invention a en outre pour objet un procédé d'aromatisation d'un produit laitier, notamment d'un fromage à pâte pressée non cuite, comprenant l'addition d'une composition d'aromatisation dans du lait.

L'addition d'une composition d'aromatisation dans du lait confère au procédé d'aromatisation d'un produit laitier un gain de temps car elle permet de réduire le temps d'affinage. En outre, elle permet de diminuer l'obtention de composés responsables de flaveurs amères indésirables ce qui limite les risques de retrait de la vente de produits laitiers ne répondant pas aux exigences des consommateurs.

Dans un mode de réalisation avantageux, la concentration en microorganismes de type lytique et en agents d'affinage est comprise entre 10⁴ et 10⁸ UFC/g de produit laitier, préférentiellement d'environ 10⁶ UFC/g de produit laitier. Typiquement, le rapport en nombre microorganismes de type lytique sur agents d'affinage pourra être compris entre 30/70 et 70/30, préférentiellement le rapport pourra être compris entre 40/60 et 60/40.

Dans un mode de réalisation particulièrement avantageux du procédé, on ajoute les microorganismes de type lytique et les agents d'affinage à raison de10⁶ à 10⁹ UFC par litre de lait, préférentiellement d'environ 10⁸ UFC par litre de lait.

Préférentiellement, le lait est d'origine animale.

Enfin, l'invention a pour objet l'utilisation d'une composition comprenant une combinaison de microorganismes, ladite combinaison comprenant au moins un microorganisme de type lytique et au moins un agent d'affinage pour l'aromatisation de produits laitiers, notamment de fromages.

L'utilisation d'une telle composition pour l'aromatisation de produits laitiers permet d'obtenir un produit laitier affiné de manière significative, c'est-à-dire significativement affiné par rapport à un procédé d'aromatisation classique. En outre, cette utilisation permet d'obtenir un produit laitier affiné ne présentant pas de mauvais goûts ou de mauvaises odeurs.

Dans un mode de réalisation avantageux, ledit agent d'affinage utilisé est préférentiellement du genre *Brevibacterium.*

Avantageusement, le produit laitier est choisi parmi les fromages à pâte molle, à pâte pressée non cuite, à pâte cuite, à pâte fraîche, à pâte persillée, les fromages fondus et « analogue cheese » (à base poudre de caséine et/ou de protéines sériques), des fromage modifiés par voie enzymatique ou « enzyme modified cheese », le Cottage cheese, ainsi que le yaourt, la crème maturée, les boissons lactées, un lait fermenté, un rétentat de produit laitier, un hydrolysat de protéines végétales, par exemple de soja, ou un lait infantile.

Encore plus avantageusement, le produit laitier est de type fromage à pâte pressée non cuite, et préférentiellement de type Gouda ou Cheddar.

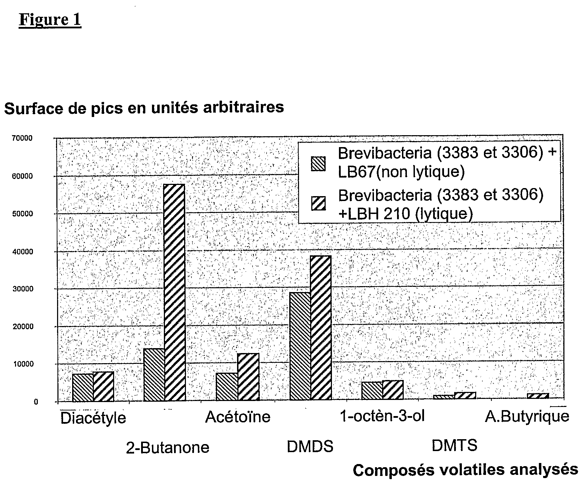

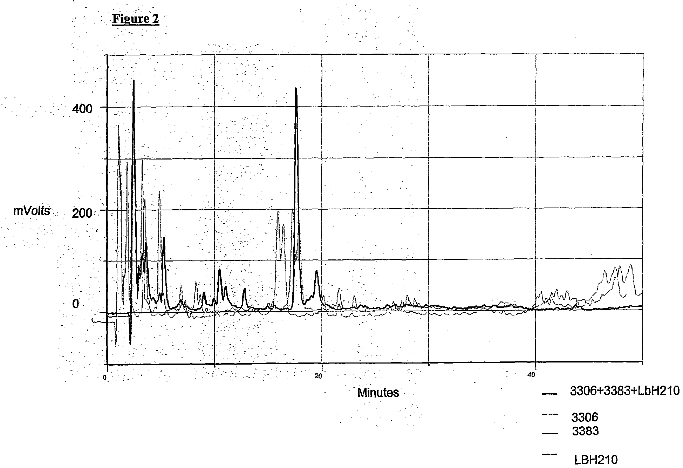

L'invention sera mieux comprise, et d'autres buts, détails, caractéristiques et avantages de celle-ci apparaîtront plus clairement au cours de la description explicative détaillée qui va suivre, de plusieurs modes de réalisation de l'invention donnés à titre d'exemples purement illustratifs et non limitatifs, en référence aux figures 1 et 2 annexées.
La figure 1 représente la comparaison de l'effet sur la production de produits aromatiques de l'utilisation d'une composition d'aromatisation selon l'invention et d'une composition d'aromatisation classique.
La figure 2 représente les chromatogrammes obtenus par HPLC des échantillons de fromage fabriqués et traités avec les souches *Brevibacterium* 3306, *Brevibacterium* 3383, et la souche *Lactobacillus helveticus* LBH 210.

L'invention concerne une composition d'aromatisation de produits laitiers, notamment de fromages, comprenant au moins une combinaison de microorganismes, ladite combinaison comprenant au moins microorganisme de type lytique et au moins un agent d'affinage.

Ledit microorganisme de type lytique est la souche *Lactobacillus helveticus* LbH 210 déposée à la CNCM le 20 janvier 2006 sous le numéro CNCM 1-3554.

Les agents d'affinage sont les bactéries du genre *Arthrobacter, Brevibacterium' Corynebacterium, Lactobacillus. Lactococcus, Leuconostoc, Micrococcus, Pediococcus, Propionibacterium, Staphylococcus et Streptococcus.* D'autres agents d'affinage connus sont des levures ou des moisissures du genre *Candida* ou *Debariomyces, Geotrichum, Kluyveromyces, Rhodotorula, Saccharomyces ou Penicilium.*

Avantageusement, ladite composition comprend en outre au moins un microorganisme du genre *Arthrobacter, Brevibacterium,* et avantageusement *Brevibacterium linens.*

Le genre *Brevibacterium* appartient à la famille Coryneformes. Les bactéries appartenant à ce genre sont des bactéries gram +, hétérofermentaires.

Dans un mode de réalisation avantageux, ledit agent d'affinage est la souche *Brevibacterium* 3306 déposée à la CNCM le 20 janvier 2006 sous le numéro CNCM 1-3556.

Dans un autre mode de réalisation avantageux, ledit agent d'affinage est la souche *Brevibacterium* 3383 déposée à la CNCM le 20 janvier 2006 sous le numéro CNCM 1-3555.

Dans un mode de réalisation particulier, lesdits microorganismes de type lytique et lesdits agents d'affinage sont en mélange ou en forme séparée.

Avantageusement, lesdits microorganismes de type lytique et lesdits agents d'affinage sont sous forme d'un mélange de souches lyophilisé ou congelé, ou sous forme de souches conditionnées de manière séparée.

La souche *Lactobacillus helveticus* LbH 210 déposée à la CNCM le 20 janvier 2006 sous le numéro CNCM 1-3554 peut également être ajoutée séparément de l'agent d'affinage.

L'utilisation d'une composition d'aromatisation de produits laitiers selon l'invention se fait de manière classique pour l'homme du métier. Dans le cas de la fabrication d'un produit laitier, celle-ci sera réalisée de manière habituelle dans ce domaine, et notamment par fermentation d'un produit laitier par incorporation d'un ferment.

Notamment, il est possible de mélanger, préalablement à l'ensemencement du milieu à fermenter, qui est en l'occurrence du lait, la combinaison comprenant au moins un microorganisme de type lytique et au moins un agent d'affinage directement avec le ferment d'acidification lactique ou grand levain.

Il est cependant possible d'incorporer séparément ou simultanément dans le milieu à fermenter, la composition d'aromatisation comprenant au moins un microorganisme de type lytique et au moins un agent d'affinage et le ferment lactique.

Les microorganismes de type lytique et les agents d'affinage peuvent être incorporés, de manière simultanée ou séparée, sous forme sèche, lyophilisée ou congelée.

La présente invention a en outre pour objet un procédé d'aromatisation d'un produit laitier, notamment d'un fromage à pâte pressée non cuite, obtenu par un procédé comprenant l'addition d'une composition d'aromatisation dans du lait.

Le produit laitier comporte du lait d'origine animale et/ou végétale.

A titre de lait d'origine animale, on peut citer le lait de vache, de chèvre, de brebis, de chamelle, de bufflesse ou leur combinaison.

A titre de lait d'origine végétale, on peut citer toute substance fermentescible d'origine végétale qui peut être utilisée selon l'invention notamment provenant des graines de soja, de riz, de coco ou de malt.

Dans un mode de réalisation particulièrement avantageux du procédé, qu'on ajoute les microorganismes de type lytique et les agents d'affinage à raison de 10⁶ à 10⁹ UFC par litre de lait, préférentiellement d'environ 10⁸ UFC par litre de lait. Typiquement la proportion de microorganismes de type lytique sera comprise entre 30 et 70% par rapport à la quantité totale d'agents d'affinage sera comprise entre Ainsi dans le produit laitier fini, la concentration des microorganismes de type lytique et des agents d'affinage est comprise entre 10⁴ et 10⁸ UFC/g de produit laitier, préférentiellement d'environ 10⁶ UFC/g de produit laitier.

Avantageusement, le produit laitier est choisi parmi les fromages à pâte molle, à pâte pressée non cuite, à pâte cuite, à pâte fraîche, à pâte persillée, les fromages fondus et « analogue cheese » (à base poudre de caséine et/ou de protéines sériques), des fromage modifiés par voie enzymatique ou « enzyme modified cheese », le Cottage cheese, ainsi que le yaourt, la crème maturée, les boissons lactées, un lait fermenté, un rétentat de produit laitier, un hydrolysat de protéines végétales, par exemple de soja, ou un lait infantile.

Encore plus avantageusement, le produit laitier et de type fromage à pâte pressée non cuite, et préférentiellement de type Gouda ou Cheddar.

Parmi les fromages, on peut citer entre autres le Banon, le Bleu d'Auvergne, le Brie, le Boulette d'Avesne, le Carphilly, le Camembert, le Cantal, le Carré de l'Est, le Chanco, le Charource, le Cheddar, le Chesire, le Cotija, le Coulommiers, le Danbo, le Dauphin, le double Gloucester, l'Edam, l'Emmenthal, l'Epoisse, la Feta, le Gorgonzola, le Gouda, le Jarisberg, le Limberger, le Livarot, la Mimolette, le Manchego, la Maroilles, le Monterey Jack, la Mozzarella, le Munster, le type Parmesan, le Pelardon, le Pont-l'Evêque, la Raclette, le Red Leicester, le Roquefort, le Saint-Félicien, le Saint-Marcellin, le Saint-Nectaire, le Saint-Paulin, le Stilton, le Tilsit, la Tomme de Savoie, le Vacherin Mont-d'Or et le Vieux-Lille.

Les exemples suivants illustrent l'invention sans toutefois en limiter la portée.

### Préparation du fromage du type Cheddar:

Un lait est reconstitué à partir de lait en poudre écrémé incorporé à 930 g pour 8.36 L d'eau. Ensuite une solution aqueuse chlorure de calcium (Calciol® de Marshall à 500 g/L d'eau) est ajoutée à une concentration de 0, 35mL/L de lait reconstitué.

Le milieu de fermentation ainsi préparé est prêt à être inoculé par des ferments d'acidification (Choozit RA073® de Danisco) et soit une composition d'aromatisation selon l'invention (comportant une souche de *Lactobacillus helveticus* de type lytique LBH 210 associé à deux souches de genre *Brevibacterium* 3383 et 3306) ou soit une composition d'aromatisation classique (comportant une souche de *Lactobacillus* de type non lytique LB 67 associé à deux souche de genre *Brevibacterium* 3383 et 3306).

Deux cuves de 10L de type Pierre Guerrin® sont ainsi utilisées.

Puis une quantité de crème est ajoutée (1000g par cuve) afin d'obtenir 33 grammes de matières grasses par litre de lait. La température est maintenue à 32°C et la vitesse d'agitation à 5 (selon la cuve de 10L de type Pierre Guerrin®).

Dans la première cuve, sont ajoutés simultanément : la souche lytique LBH 210 à raison de 1×10⁸UFC/L de milieu de fermentation, les souches 3383 et 3306 à raison de 1×10⁸UFC/L de milieu de fermentation et un ferment d'acidification classique (Choozit RA073® de Danisco) à raison de 2×10⁹UFC/L de milieu de fermentation.

Dans la seconde cuve, sont ajoutés simultanément : la souche non lytique LB67 (déposée à la CNCM le 20 janvier 2006 sous le numéro CNCM 1-3553), à raison de 2×10⁸UFC/L de milieu de fermentation, les souches 3383 et 3306 à raison de 1×10⁸UFC/L de milieu de fermentation et un ferment d'acidification classique (Choozit RA073® de Danisco) à raison de 2×10⁹UFC/L de milieu de fermentation.

Puis dans chacune des cuves, le mélange est ensuite soumis à maturation pendant 45 minutes sous agitation (vitesse 5).

De la présure, ayant une concentration de 520 mg de chymosine par litre de présure, est ajoutée dans le mélange après maturation à raison de 25mL par 100 litre de lait. Le pH est enregistré au cours de la fabrication à l'aide d'un pH-mètre. La coagulation se déroule pendant 20 à 30 minutes.

Après coagulation, le caillé obtenu est laissé au repos 20 minutes et est ensuite découpé. Le découpage se fait longitudinalement et transversalement. L'ensemble formé du caillé et du sérum, est chauffé de 32°C à 42°C et soumis à une agitation de vitesse 5 pendant 120 minutes.

Le sérum qui se forme est soutiré de manière continue.

Le caillé de chacune des cuves est placé dans deux moules de 14 cm de diamètre.

Puis le caillé de chacun des moules est découpé en pièces égales (4 à 8) environ toutes les 15 minutes pour soutirer du sérum, puis retourné.

A partir du moment où le pH de chaque caillé atteint 5,2, chaque caillé est découpé en petits morceaux (de moins d'un centimètre de diamètre) et 3% de NaCl en masse par rapport à la masse du caillé est ajouté. Puis chaque caillé est mélangé et moulé dans un moule de 14 cm de diamètre et pressé successivement à différentes pressions. La pression exercée initialement est de 1,5 bars pendant 30 minutes puis cette pression est augmentée toutes les 30 minutes de 0,5 bar. Enfin, la pression de 3 bars est maintenue toute une nuit.

24 heures après l'inoculation, chaque caillé moulé est pesé et emballé en film sous vide, de manière imperméable.

Puis les fromages sont stockés à 4°C pendant environ une semaine.

Ensuite, des échantillons de fromage sont soumis à une procédure d'affinage accélérée par mise en suspension (« slurrying »), selon la méthode IBT standard method PM 4.0. Cette méthode est utilisée de façon à comprendre dans une échelle de temps réduite à semaine (au lieu de trois mois pour un affinage classique), les mécanismes biochimiques de l'affinage. Dans ces conditions les processus enzymatiques impliqués dans l'affinage sont accélérés.

Pour cela, un échantillon de 100g fromage (fabriqué avec une composition d'aromatisation selon l'invention ou une composition d'aromatisation classique) est broyé dans 50mL d'eau en présence de 1g de citrate de trisodium et de 300mg d'acide ascorbique. Chaque mélange est ensuite mis sous vide pendant une semaine à 30°C de façon à obtenir une suspension dite finale.

Puis des essais ont été effectués sur la suspension finale obtenue.

Le premier essai consiste en quantifier la présence d'acide aminés dans chacune des suspensions.

Les résultats sont donnés dans le tableau 1 suivant.

**Tableau 1 : Comparaison de la quantité d'acides aminés obtenue avec des compositions d'affinage différentes :**

| | *Brevibacterium* 3306 et 3383 + *Lactobacillus helveticus* LB 67 | *Brevibacterium* 3306 et 3383 + *Lactobacillus helveticus* LBH 210 |
|---|---|---|
| Quantité d'acides amines en mg/g de suspension finale | 10,2 | 10,9 |

En présence de microorganismes lytiques, on obtient une plus grande quantité d'acides aminés et donc une plus grande flaveur, les acides aminés étant fortement impliqués dans le développement de la flaveur d'un produit laitier.

Ensuite, un échantillon de chacune des suspensions finales (obtenue avec une composition d'aromatisation selon l'invention ou avec une composition d'aromatisation classique) est analysé par chromatographie en phase gazeuse (CPG) de façon à déterminer les composés volatiles impliqués dans le développement de la flaveur d'un produit laitier.

### Protocole de la CPG :

Dans un flacon à sertir de 10g sont introduits : 3g chlorure de sodium Normapur à 99,5%, 5g d'échantillon à analyser (dans le cas présent 5g de suspension dite « finale », et 2 mL d'eau ultra pure.

L'extraction est réalisée par un dispositif de type Headspace HS40XL® de Perkin-Elmer. Le temps de chauffage appliquée est de 30 minutes, la température de l'échantillon de 60°C, la température de l'aiguille de 80°C et la température de transfert de 100°C.

La séparation et le dosage est effectuée par un dispositif de type CPG Autosystème XL® de Perkin-Elmer. La colonne utilisée est de type CP-SIL5CB (Varian®), de type Wcot fused silice 30m×0,32mm (diamètre interne).

La phase stationnaire est constituée de 100% de diméthylpolysiloxane. L'épaisseur du film est de 0,5µm.

Le gaz vecteur est de l'hélium de débit égal à 1,6mL/minute.

Le cycle appliqué est le suivant : 40°C pendant 2 minutes puis 10°C jusqu'à 160°C, puis 160°C pendant 3 minutes.

Le détecteur est un détecteur de type à ionisation de flamme. La température maximale appliquée est de 250°C.

Les résultats sont présentés par la figure 1.

La surface des pics de chromatographie obtenue est quantifiée selon des unités arbitraires qui sont représentées sur l'axe des ordonnées du graphique de la figure 1.

L'ensemble des composés volatiles suivants : c'est-à-dire le diacétyle, le 2-butanone, l'acétoïne, le DMDS, le 1-octén-3-ol, le DMTS et l'acide butyrique composé volatile sont présents en quantités significativement plus importantes lorsqu'une composition incorporant une souche de *Lactobacillus helveticus* LBH 210 (souche lytique) combinée à deux souches de *Brevibacterium* est utilisée que lorsqu'une composition incorporant une souche de *Lactobacillus helveticus* LB 67 (souche non lytique) combinée à deux souche de *Brevibacterium* est utilisée. Ces résultats sont d'autant plus significatifs qu'il a été incorporé une quantité deux fois plus importante de *Lactobacillus helveticus* non lytique que de *Lactobacillus helveticus* lytique comparativement dans les échantillons.

Enfin, différents échantillons de fromage sont analysés par chromatographie en phase liquide en phase inverse de façon à déterminer les composés non volatiles impliqués dans le développement de la flaveur d'un produit laitier.

Les différents échantillons de fromage sont issus de fromages fabriqués soit avec une composition d'aromatisation comportant *Lactobacillus helveticus* LBH 210 (souche lytique) en présence de *Brevibacterium* 3383 et 3306, soit *Lactobacillus helveticus* LBH 210 (souche lytique) uniquement, soit *Brevibacterium* 3383 uniquement, ou soit *Brevibacterium* 3383 uniquement.

### Protocole de l'HPLC utilisée :

5g de fromage de chaque échantillon préalablement pesé sont broyés au Valentin dans un tube de 50ml. Le fait de broyer chaque échantillon permet d'optimiser l'extraction et d'avoir un échantillon homogène.

Puis 20mL de tampon citrate pH3.0 est ajouté au fromage broyé. L'échantillon est ensuite homogénéisé à l'Ultra-Turrax pendant 20 secondes. L'opération est renouvelée jusqu'à obtention d'un mélange homogène. Puis le tube est ensuite placé dans l'étuve à 40°C pendant 1 heure. Après cette étape, la matière grasse se trouve en surface. Afin de mieux l'extraire, il est conseillé de placer les tubes dans la glace afin de figer la phase grasse.

Une fois la phase grasse extraite, l'échantillon est centrifugé à 3000rpm pendant 35 minutes.

Après centrifugation, les peptides se retrouvent dans le surnageant.

Pour effectuer l'analyse par HPLC, il faut prélever 1ml de surnageant, le placer dans un tube Eppendorf® de 1,5ml et centrifuger à 13000rpm (tour par minutes) pendant 10minutes.

Après centrifugation, 1ml de surnageant est prélevé à l'aide d'une seringue à filtration et filtré à travers un filtre Acrodisc Nylon 13-0.45µm Récupérer le filtrat dans un tube à HPLC et sertir. L'échantillon est alors prêt à être analysé par chromatographie haute performance en phase liquide inverse (reverse phase HPLC) par l'utilisation d'un gradient Eau/Acetonitrile sur une durée de 80 minutes avec l'utilisation d'une colonne Phenomenex Jupiter® 10µm C18 de 300Å 250x4.6mm.

En outre, des échantillons de fromage fabriqués et traités de manière analogue que les échantillons évoqués précédemment, à l'exception près que les microorganismes inoculés simultanément avec ferment d'acidification sont soit des souches *Brevibacterium* 3306, soit des souches *Brevibacterium* 3383, ou encore soit des souches *Lactobacillus helveticus* LBH 210.

Les résultats sont présentés sur la figure 2 et le tableau 2 suivant.

**Tableau 2 : Comparaison de la quantité de certains composés non volatiles impliqués dans la flaveur du fromage de type Cheddar dans différents échantillons :**

| | Surface de pics en unités arbitraires | | | |
|---|---|---|---|---|
| | 3306 | 3383 | LbH210 | 3306+3383+ LbH210 |
| Elution à environ 17 minutes | 17,594,796 (2 pics) | 17,409,966 (2 pics) | 1,213,530 (2 pics) | 16,662,131 (1 pic) |
| Elution à environ 18 minutes | 272,259 | 312,993 | 451,919 | 4,650,971 |
| Elution entre 45 et 51 minutes | 28,910,454 | 36,486,252 | 10,075,686 | 2,397,232 |

Il a été obtenu que, en comparaison avec l'utilisation des souches isolément, avec l'utilisation de la combinaison des souches *Brevibacterium* 3306 + *Brevibacterium* 3383 + *Lactobacillus helveticus* LBH 210 :
- on obtient une forte diminution de peptides de grande taille hydrophobes (dont le temps d'élution est compris entre 45 et 51 minutes sur la figure 2). Ces peptides confèrent généralement de l'amertume au produit laitier et sont à éviter,
- on obtient une quantité significative de peptides de petite taille, hydrophiles, dont la nature est modifiée (on obtient un seul pic à environ 17 minutes dont l'aire est équivalente à la somme des aires deux pics obtenus lors de l'utilisation séparée des souches d'affinage, sur la figure 2). Ces peptides confèrent généralement des flaveurs appréciées dans les produits laitiers (tels que le goût de bouillon de viande ou des goûts savoureux par exemple) et
- on obtient l'apparition de peptides de petite taille hydrophiles non présents lors de l'utilisation des souches isolément (dont le temps d'élution est à environ 18 minutes sur la figure 2). Ces peptides appartiennent à la gamme des peptides qui confèrent généralement des flaveurs appréciées dans les produits laitiers et permettent de varier ou affiner encore la flaveur obtenue lors de l'affinage.

## Revendications

1. Composition d'aromatisation de produit laitiers comprenant la souche *Lactobacillus helveticus* LbH 210 déposée à la CNCM le 20 janvier 2006 sous le numéro CNCM 1-3554 et au moins un agent d'affinage choisi parmi les bactéries du genre *Arthrobacter, Brevibacterium, Corynebacterium, Lactobacillus, Lactococcus, Leuconostoc, Micrococcus, Pediococcus, Propionibacterium, Staphylococcus et Streptococcus.*

2. Combinaison pour l'aromatisation de produits laitiers comprenant, conditionnés de manière séparée, la souche *Lactobacillus helveticus* LbH 210 déposée à la CNCM le 20 janvier 2006 sous le numéro CNCM 1-3554 et au moins un agent d'affinage choisi parmi les bactéries du genre *Arthrobacter, Brevibacterium, Corynebacterium, Lactobacillus, Lactococcus, Leuconostoc, Micrococcus, Pediococcus, Propionibacterium, Staphylococcus et Streptococcus.*

3. Composition selon la revendication 1 ou combinaison selon la revendication 2, **caractérisé en ce que** le au moins un agent d'affinage est un microorganisme du genre *Arthrobacter* ou *Brevibacterium.*

4. Composition ou combinaison selon l'une quelconque des revendications précédentes, **caractérisé en ce que** le au moins un agent d'affinage est de l'espèce *Brevibacterium linens.*

5. Composition ou combinaison selon l'une quelconque des revendications précédentes, **caractérisé en ce que** ledit au moins un agent d'affinage est la souche *Brevibacterium* 3306 déposée à la CNCM le 20 janvier 2006 sous le numéro CNCM 1-3556.

6. Composition ou combinaison selon l'une quelconque des revendications précédentes, **caractérisé en ce que** ledit au moins un agent d'affinage est la souche *Brevibacterium* 3383 déposée à la CNCM le 20 janvier 2006 sous le numéro CNCM 1-3555.

7. Composition selon l'une quelconque des revendications 1 et 3 à 6, **caractérisée en ce que** la souche *Lactobacillus helveticus* LbH 210 déposée à la CNCM le 20 janvier 2006 sous le numéro CNCM 1-3554 et ledit au moins un agent d'affinage sont sous forme d'un mélange de souches lyophilisé ou congelé.

8. Souche *Lactobacillus helveticus* LbH 210 déposée à la CNCM le 20 janvier 2006 sous le numéro CNCM 1-3554.

9. Souche *Lactobacillus helveticus* LbH 210 déposée à la CNCM le 20 janvier 2006 sous le numéro CNCM 1-3554 en combinaison avec des ferments d'acidification de type *Lactococcus lactis* et/ou *Streptococcus thermophilus.*

10. Procédé d'aromatisation d'un produit laitier, notamment d'un fromage à pâte pressée non cuite, comprenant l'addition d'une composition ou d'une combinaison selon l'une quelconque des revendications 1 à 7, dans du lait.

11. Procédé selon la revendication 10, **caractérisé en ce que** la concentration en microorganismes de type lytique et en agents d'affinage est comprise entre 10⁴ et 10⁸ UFC/g de produit laitier, préférentiellement d'environ 10⁶ UFC/g de produit laitier.

12. Procédé selon la revendication 10 ou 11, **caractérisé en ce qu'**on ajoute la souche *Lactobacillus helveticus* LbH 210 déposée à la CNCM le 20 janvier 2006 sous le numéro CNCM 1-3554 et les agents d'affinage à raison de 10⁶ à 10⁹ UFC par litre de lait, préférentiellement d'environ 10⁸ UFC par litre de lait.

13. Procédé selon l'une des revendications 10 à 12, **caractérisé en ce que** le lait est d'origine animale.

14. Procédé selon l'une quelconque des revendications 10 à 13, **caractérisé en ce que** la souche *Lactobacillus helveticus* LbH 210 déposée à la CNCM le 20 janvier 2006 sous le numéro CNCM 1-3554 et les agents d'affinage sont ajoutés de manière simultanée ou séparée.

15. Utilisation d'une composition de microorganismes comprenant au moins la souche *Lactobacillus helveticus* LbH 210 déposée à la CNCM le 20 janvier 2006 sous le numéro CNCM 1-3554 et au moins un agent d'affinage choisi parmi les bactéries du genre *Arthrobacter, Brevibacterium, Corynebacterium, Lactobacillus, Lactococcus, Leuconostoc, Micrococcus, Pediococcus, Propionibacterium, Staphylococcus et Streptococcus* pour l'aromatisation de produits laitiers, notamment de fromages.

16. Utilisation d'une combinaison comprenant, conditionnés de manière séparée, au moins la souche *Lactobacillus helveticus* LbH 210 déposée à la CNCM le 20 janvier 2006 sous le numéro CNCM 1-3554 et au moins un agent d'affinage choisi parmi les bactéries du genre *Arthrobacter, Brevibacterium, Corynebacterium, Lactobacillus, Lactococcus, Leuconostoc, Micrococcus, Pediococcus, Propionibacterium, Staphylococcus et Streptococcus* pour l'aromatisation de produits laitiers, notamment de fromages.

17. Utilisation selon la revendication 15 ou 16, **caractérisée en ce que** ledit au moins un agent d'affinage est du genre *Brevibacterium.*

18. Utilisation selon l'une quelconque des revendications 15 à 17, **caractérisée en ce que** le produit laitier est choisi parmi les fromages à pâte molle, à pâte pressée non cuite, à pâte cuite, à pâte fraîche, à pâte persillée, les fromages fondus et « analogue cheese », des fromage modifiés par voie enzymatique, le Cottage cheese, ainsi que le yaourt, la crème maturée, les boissons lactées, un lait fermenté, un rétentat de produit laitier, un hydrolysat de protéines végétales ou un lait infantile.

19. Utilisation selon la revendication 18, **caractérisée en ce que** le produit laitier est de type fromage à pâte pressée non cuite, et préférentiellement de type Gouda ou Cheddar.

## Patentansprüche

1. Zusammensetzung für die Aromatisierung von Milchprodukten umfassend den Stamm *Lactobacillus helveticus* LbH 210, angemeldet bei der CNCM am 20. Januar 2006 unter der Nummer CNCM 1-3554 und mindestens ein Verfeinerungsmittel, ausgewählt unter den Bakterien der Spezies *Arthrobacter, Brevibacterium, Corynebacterium, Lactobacillus, Lactococcus, Leuconostoc, Micrococcus, Pediococcus, Propionibacterium, Staphylococcus* und *Streptococcus.*

2. Zusammensetzung für die Aromatisierung von Milchprodukten umfassend, separat verpackt, den Stamm *Lactobacillus helveticus* LbH 210, angemeldet bei der CNCM am 20. Januar 2006 unter der Nummer CNCM 1-3554 und mindestens ein Verfeinerungsmittel, ausgewählt unter den Bakterien der Spezies *Arthrobacter, Brevibacterium, Corynebacterium, Lactobacillus, Lactococcus, Leuconostoc, Micrococcus, Pediococcus, Propionibacterium, Staphylococcus* und *Streptococcus.*

3. Zusammensetzung nach Anspruch 1 oder Kombination nach Anspruch 2, **dadurch gekennzeichnet, dass** das mindestens eine Verfeinerungsmittel ein Mikroorganismus der Spezies *Arthrobacter* oder *Brevibacterium* ist.

4. Zusammensetzung oder Kombination nach einem der vorherigen Ansprüche, **dadurch gekennzeichnet, dass** das mindestens eine Verfeinerungsmittel von der Spezies *Brevibacterium linens* ist.

5. Zusammensetzung oder Kombination nach einem der vorherigen Ansprüche, **dadurch gekennzeichnet, dass** das mindestens eine Verfeinerungsmittel der Stamm *Brevibacterium* 3306 ist, angemeldet bei der CNCM am 20. Januar 2006 unter der Nummer CNCM I-3556.

6. Zusammensetzung oder Kombination nach einem der vorherigen Ansprüche précédentes, **dadurch gekennzeichnet, dass** das mindestens eine Verfeinerungsmittel der Stamm *Brevibacterium* 3383 ist, angemeldet bei der CNCM am 20. Januar 2006 unter der Nummer CNCM I-3555.

7. Zusammensetzung nach einem der Ansprüche 1 und 3 bis 6, **dadurch gekennzeichnet, dass** der Stamm *Lactobacillus helveticus* LbH 210, angemeldet bei der CNCM am 20. Januar 2006 unter der Nummer CNCM 1-3554 und das mindestens eine Verfeinerungsmittel in Form eines Gemischs aus gefriergetrockneten oder gefrorenen Stämmen ist.

8. Stamm *Lactobacillus helveticus* LbH 210, angemeldet bei der CNCM am 20. Januar 2006 unter der Nummer CNCM I-3554.

9. Stamm *Lactobacillus helveticus* LbH 210, angemeldet bei der CNCM am 20. Januar 2006 unter der Nummer CNCM 1-3554 in Kombination mit Säuerungsfermenten vom Typ *Lactococcus lactis* und/oder *Streptococcus thermophilus.*

10. Verfahren für die Aromatisierung eines Milchprodukts, besonders eines Käses aus nicht gekochter gepresster Paste, umfassend das Hinzufügen einer Zusammensetzung oder einer Kombination nach einem der Ansprüche 1 bis 7 in die Milch.

11. Verfahren nach Anspruch 10, **dadurch gekennzeichnet, dass** die Konzentration an Mikroorganismen vom Typ lytisch und an Verfeinerungsmitteln zwischen 10⁴ und 10⁸ UFC/g Milchprodukt liegt, vorzugsweise 10⁶ UFC/g Milchprodukt.

12. Verfahren nach Anspruch 10 oder 11, **dadurch gekennzeichnet, dass** der Stamm *Lactobacillus helveticus* LbH 210, angemeldet bei der CNCM am 20. Januar 2006 unter der Nummer CNCM 1-3554, und die Verfeinerungsmittel mit 10⁶ bis 10⁹ UFC pro Liter Milch, vorzugsweise mit ca. 10⁸ UFC pro Liter Milch hinzugefügt werden.

13. Verfahren nach einem der Ansprüche 10 bis 12, **dadurch gekennzeichnet, dass** die Milch tierischen Ursprungs ist.

14. Verfahren nach einem der Ansprüche 10 bis 13, **dadurch gekennzeichnet, dass** der Stamm *Lactobacillus helveticus* LbH 210, angemeldet bei der CNCM am 20. Januar 2006 unter der Nummer CNCM 1-3554 und die Verfeinerungsmittel gleichzeitig oder separat hinzugefügt werden.

15. Verwendung einer Zusammensetzung von Mikroorganismen umfassend mindestens den Stamm *Lactobacillus helveticus* LbH 210, angemeldet bei der CNCM am 20. Januar 2006 unter der Nummer CNCM 1-3554 und mindestens ein Verfeinerungsmittel, ausgewählt unter den Bakterien der Spezies *Arthrobacter, Brevibacterium, Corynebacterium, Lactobacillus, Lactococcus, Leuconostoc, Micrococcus, Pediococcus, Propionibacterium, Staphylococcus* und *Streptococcus* für die Aromatisierung von Milchprodukten, besonders von Käsen.

16. Verwendung einer Kombination umfassend, separat verpackt, mindestens den Stamm *Lactobacillus helveticus* LbH 210, angemeldet bei der CNCM am 20. Januar 2006 unter der Nummer CNCM 1-3554 und mindestens ein Verfeinerungsmittel, ausgewählt unter den Bakterien der Spezies *Arthrobacter, Brevibacterium, Corynebacterium, Lactobacillus, Lactococcus, Leuconostoc, Micrococcus, Pediococcus, Propionibacterium, Staphylococcus* und *Streptococcus* für die Aromatisierung von Milchprodukten, besonders von Käsen.

17. Verwendung nach Anspruch 15 oder 16, **dadurch gekennzeichnet, dass** das mindestens eine Verfeinerungsmittel von der Spezies *Brevibacterium* ist.

18. Verwendung nach einem der Ansprüche 15 bis 17, **dadurch gekennzeichnet, dass** das Milchprodukt ausgewählt ist von den Käsen aus weicher Paste, aus nicht gekochter gepresster Paste, aus gekochter Paste, aus frischer Paste, aus Paste mit Petersilie, den Schmelzkäsen und "analogem Cheese", enzymatisch modifizierten Käsen, dem Cottage Cheese, sowie Jogurt, gereifter Creme, den Milchgetränken, einer fermentierten Milch, einem Retentat des Milchprodukts, einem Hydrolysat aus pflanzlichen Proteinen oder einer Kindermilch.

19. Verwendung nach Anspruch 18, **dadurch gekennzeichnet, dass** das Milchprodukt vom Typ eines Käses aus nicht gekochter gepresster Paste ist, und vorzugsweise vom Typ Gouda oder Cheddar.

## Claims

1. Composition for flavouring dairy products comprising the strain *Lactobacillus helveticus* LbH 210 filed at the CNCM on 20th January 2006 under the number CNCM I-3554 and at least one refining agent which is selected from the bacteria of the genus *Arthrobacter, Brevibacterium, Corynebacterium, Lactobacillus, Lactococcus, Leuconostoc, Micrococcus, Pediococcus, Propionibacterium, Staphylococcus* and *Streptococcus.*

2. Combination for flavouring dairy products comprising, in a state processed separately, the strain *Lactobacillus helveticus* LbH 210 filed at the CNCM on 20th January 2006 under the number CNCM 1-3554 and at least one refining agent which is selected from the bacteria of the genus *Arthrobacter, Brevibacterium, Corynebacterium, Lactobacillus, Lactococcus, Leuconostoc, Micrococcus, Pediococcus, Propionibacterium, Staphylococcus* and *Streptococcus.*

3. Composition according to claim 1 or combination according to claim 2, **characterised in that** the at least one refining agent is a microorganism of the genus *Arthrobacter* or *Brevibacterium.*

4. Composition or combination according to any one of the preceding claims, **characterised in that** the at least one refining agent is of the species *Brevibacterium linens.*

5. Composition or combination according to any one of the preceding claims, **characterised in that** the at least one refining agent is the strain *Brevibacterium* 3306 filed at the CNCM on 20th January 2006 under the number CNCM I-3556.

6. Composition or combination according to any one of the preceding claims, **characterised in that** the at least one refining agent is the strain *Brevibacterium* 3383 filed at the CNCM on 20th January 2006 under the number CNCM I-3555.

7. Composition according to any one of claims 1 and 3 to 6, **characterised in that** the strain *Lactobacillus helveticus* LbH 210 filed at the CNCM on 20th January 2006 under the number CNCM 1-3554 and the at least one refining agent are in the form of an admixture of lyophilised or frozen strains.

8. Strain *Lactobacillus helveticus* LbH 210 filed at the CNCM on 20th January 2006 under the number CNCM I-3554.

9. Strain *Lactobacillus helveticus* LbH 210 filed at the CNCM on 20th January 2006 under the number CNCM 1-3554 in combination with acidification enzymes of the type *Lactococcus lactis* and/or *Streptococcus thermophilus.*

10. Method for flavouring a dairy product, in particular an uncooked pressed cheese, comprising the addition of a composition or a combination according to any one of claims 1 to 7 to milk.

11. Method according to claim 10, **characterised in that** the concentration of microorganisms of the lytic type and refining agents is between 10⁴ and 10⁸ CFU/g of dairy product, preferably approximately 10⁶ CFU/g of dairy product.

12. Method according to claim 10 or 11, **characterised in that** the strain *Lactobacillus helveticus* LbH 210 filed at the CNCM on 20th January 2006 under the number CNCM I-3554 and the refining agents are added at a ratio of from 10⁶ to 10⁹ CFU per litre of milk, preferably approximately 10⁸ CFU per litre of milk.

13. Method according to any one of claims 10 to 12, **characterised in that** the milk is of animal origin.

14. Method according to any one of claims 10 to 13, **characterised in that** the strain *Lactobacillus helveticus* LbH 210 filed at the CNCM on 20th January 2006 under the number CNCM 1-3554 and the refining agents are added simultaneously or separately.

15. Use of a composition of microorganisms comprising at least the strain *Lactobacillus helveticus* LbH 210 filed at the CNCM on 20th January 2006 under the number CNCM 1-3554 and at least one refining agent which is selected from the bacteria of the genus *Arthrobacter, Brevibacterium, Corynebacterium, Lactobacillus, Lactococcus, Leuconostoc, Micrococcus, Pediococcus, Propionibacterium, Staphylococcus* and *Streptococcus* for flavouring dairy products, in particular cheeses.

16. Use of a combination comprising, in a state processed separately, at least the strain *Lactobacillus helveticus* LbH 210 filed at the CNCM on 20th January 2006 under the number CNCM 1-3554 and at least one refining agent which is selected from the bacteria of the genus *Arthrobacter, Brevibacterium, Corynebacterium, Lactobacillus, Lactococcus, Leuconostoc, Micrococcus, Pediococcus, Propionibacterium, Staphylococcus* and *Streptococcus* for flavouring dairy products, in particular cheeses.

17. Use according to claim 15 or 16, **characterised in that** the at least one refining agent is of the genus *Brevibacterium.*

18. Use according to any one of claims 15 to 17, **characterised in that** the dairy product is selected from soft cheeses, uncooked pressed cheeses, cooked cheeses, fresh cheeses, veined cheeses, molten cheeses and "analogue cheeses", cheeses which are modified by means of enzymes, cottage cheese, and yoghurt, cured cream, dairy drinks, a fermented milk, a dairy product retentate, a hydrolysate of plant protein or infant milk.

19. Use according to claim 18, **characterised in that** the dairy product is of the uncooked pressed cheese type, and preferably of the Gouda or Cheddar type.
